# EUROPEAN PATENT APPLICATION

(11) **EP 0 653 493 A1**
(43) Date of publication of application: **17.05.1995**
(21) Application number: 93911957.4
(22) Date of filing: 30.04.1993
(51) Int. Cl.: C12Q 1/68, C12N 15/00

(54) **METHOD OF DETECTING EXPRESSION OF T-CELL ANTIGEN RECEPTOR GENE**

(30) Priority: 30.04.1992 JP 111467/92; 31.07.1992 JP 205054/92
(71) Applicant: TAISHO PHARMACEUTICAL CO. LTD, Tokyo 171 (JP); LTT INSTITUTE CO., LTD., Kawasaki-shi, Kanagawa 216 (JP)
(72) Inventor: YAMAMOTO, Kazuhiko, Kita-ku, Tokyo 114 (JP); MIZUSHIMA, Yutaka, Setagaya-ku, Tokyo 154 (JP); NISHIOKA, Kusuki, Shibuya-ku, Tokyo 150 (JP); SAKODA, Hiroko, Toshima-ku, Tokyo 171 (JP); IKEDA, Yoko, Toshima-ku, Tokyo 171 (JP)
(74) Representative: Ablewhite, Alan James
(86) International application number: JP9300577
(87) International publication number: WO9322455

(57) **Abstract**

A method of detecting the presence of a T-cell antigen receptor gene which is specific for T-cell clones accumulated in a specimen, which method comprises preparing a cDNA from T-cells in a specimen, amplifying a T-cell antigen receptor gene in the cDNA by the PCR method, and analyzing the amplified gene by the single-strand conformation polymorphism (SSCP) method.

## Description

### TECHNICAL FIELD

The present invention relates to a method for detecting expression of T-cell antigen receptor (referred to as T cell receptor hereinafter) genes. More particularly, the present invention relates to a method for detecting the presence of a gene specific to T cell clone accumulated in a test sample which comprises analyzing a β chain of T-cell receptor genes in T cells contained in the test sample, by applying a polymerase chain reaction (PCR) and a single strand conformation polymorphism (SSCP) technique.

### BACKGROUND ART

Immune response which is a protective mechanism of an organism is roughly classified into humoral immunity and cell-mediated or cellular immunity; in humoral immunity, a B cell-producing specific antibody is reactive with an antigen and in cell-mediated immunity, T cells release an active substance or act directly on antigen-presenting cells.

T cells are responsible for cellular immunity and recognize antigens; not only antigens but histocompatibility antigens on antigen-presenting cells such as macrophages, B cells or dendritic cells are required for recognition by T cells. T cell receptors present on T cells recognize a complex of this histocompatibility antigen and a foreign antigen in antigen-presenting cells.

The T cell receptor is a heterodimer of α and β or γ and δ consisting of two pairs of disulfide-linked polypeptide chains and is expressed on cell membrane as a complex with T3 (CD₃) antigen. Each chain of α, β, γ and δ contain the variable region (V domain) which differs in response to the antigenic specificity of each T cell and the constant region (C domain) which has the same sequence. The α and γ chains further have J (joining) segment between the V and C domains and the β and δ chains further have D (diversity) and J (joining) segments between the V and C domains.

Little is hitherto known about the relationship between pathogenic antigens of an autoimmune disease such as chronic rheumatoid arthritis and antigen-specific T cells. However, there are reports implying that an accumulation of some clonal T cells or of T cells expressing certain V genes of T cell receptors (Stamenkovic, I. et al., Proc. Natl. Acad. Sci. USA, 85: 1179-1183 (1988); Hakoda, M. et al., Clin. Immunol. Immunopathol., 57: 387-398 (1990)). On the other hand, it is also reported that no significant clonal expansion of specific T cells have been noted in patient with chronic rheumatoid arthritis but polyclonal T cell activation would dominate (Keystone, E. C. et al., Arthritis Rheum., 31: 1555-1557 (1988); Duby, A. D. et al., Proc. Natl. Acad. Sci. USA, 86: 6209-6210 (1989); Oksenkerg, J. R. et al., Nature, 345: 344-346 (1990)). Similar conflicting data have also been reported in studies of other diseases.

Such conflict probably resulted from difficulties both in detailed analysis on T cell receptor genes expressed in a particular cell mass and in analysis of genes in the D and J segments of β chain.

### DISCLOSURE OF INVENTION

Therefore, an object of the present invention is to provide a method for detecting a T-cell clone accumulated in a test sample and for detecting the presence of a T cell receptor gene expressed by the T cell clone, which also enables to genetically analyze the D and J regions of β chain in the T-cell receptors.

The present inventor have made extensive studies on a method for detection which enables to analyze T cell receptor genes in detail. As a result, it has been found that by applying a polymerase chain reaction (PCR) and a single strand conformation polymorphism (SSCP) technique to the analysis of T-cell receptor genes, diversities of genes in the D and J regions of the β chain can be analyzed and the presence of T cell receptor genes in which a T cell clone accumulated in a test sample is expressed, can be detected. The present invention has thus been attained.

Therefore, the present invention relates to a method for detecting the presence of T cell receptor genes which comprises:
preparing a cDNA mixture comprising T cell-derived cDNA from a test sample containing T cells;
subjecting the cDNA mixture to polymerase chain reaction (PCR) using as a primer a DNA fragment encoding the β chain of T-cell receptors to amplify the genes encoding the β chain of the T-cell receptors in the cDNA mixture; and then,
subjecting the thus amplified genes to analysis by the single strand conformation polymorphism (SSCP) technique to detect the presence of the T cell clone-specific genes accumulated in the test sample which encodes the β chain of the T-cell receptors.

### BRIEF DESCRIPTION OF DRAWINGS

Fig. 1 shows the results obtained by detecting peripheral blood collected from healthy individuals according to the method of the present invention.

Fig. 2 shows the results obtained by detecting human T cell tumor line HPB-ALL according to the method of the present invention.

Fig. 3 shows the results obtained by detecting a mixture of peripheral blood collected from healthy individuals and HPB-ALL according to the method of the present invention.

Fig. 4 shows the results obtained by detecting peripheral blood collected from healthy individuals by applying various antigenic stimulations, according to the method of the present invention.

Fig. 5 shows the results obtained by detecting a sample obtained from a patient with chronic rheumatoid arthritis according to the method of the present invention.

Fig. 6 shows the results obtained by detecting a sample obtained from a patient with chronic rheumatoid arthritis according to the method of the present invention.

Fig. 7 shows the results obtained by detecting a sample obtained from a patient with chronic rheumatoid arthritis according to the method of the present invention.

Fig. 8 shows the results obtained by detecting a sample obtained from a patient with chronic rheumatoid arthritis according to the method of the present invention.

Fig. 9 shows the results obtained by detecting a sample obtained from a patient with chronic rheumatoid arthritis according to the method of the present invention.

Fig. 10 shows the results obtained by detecting a sample obtained from a patient with uterus cancer according to the method of the present invention.

Fig. 11 shows the results obtained by detecting a sample obtained from a patient with uterus cancer according to the method of the present invention.

Fig. 12 shows the results obtained by detecting a sample obtained from a patient with sarcoidosis according to the method of the present invention.

Fig. 13 shows the results obtained by detecting a sample obtained from a patient with hepatitis C according to the method of the present invention.

Fig. 14 shows the results obtained by detecting a sample obtained from a patient with chronic rheumatoid arthritis, using hybridization technique according to the method of the present invention.

Fig. 15 shows the results obtained by detecting a sample obtained from a patient with chronic rheumatoid arthritis, using hybridization technique according to the method of the present invention.

### BEST MODE FOR CARRYING OUT THE INVENTION

The test sample used in the present invention is a specimen containing T cells therein. Specifically, the test sample refers to peripheral blood, tissue fragments obtained from patients who are suspected of abnormal immune response or from patients with viral diseases, or peripheral blood or tissue fragments from patients with cancer who can expect immune effects. Acceptable test samples which can be used for the method of the present invention depend upon the disease and typically include peripheral blood, synovial fluid or synovial tissues in the case of patients with chronic rheumatoid arthritis; peripheral blood, afflicted lymph nodes or cancer focus in the case of patients with cancer; peripheral blood or bronchoalveolar lavage fluids in the case of patients with sarcoidosis; and peripheral blood or liver biopsy sample in the case of patients with hepatitis.

From these samples, a mixture of cDNAs containing T cell-derived cDNAs is prepared.

As an embodiment, T lymphocytes are separated from peripheral blood or synovial fluid in a conventional manner to isolate RNAs or, synovial tissues are homogenized to isolate RNAs. Alternatively, RNAs are similarly isolated from bronchoalveolar lavage fluids which is the focus of a patient with sarcoidosis, or a liver biopsy sample which is the focus of a patient with hepatitis C.

RNAs can be isolated in a conventional manner by applying thereto, e.g., the AGPC method (Chomczynski, P. & Sacchi, N., Anal. Biochem., 162: 152-159 (1987)). Using a reverse transcriptase and a random primer, the cDNA mixture is then obtained from RNAs.

The cDNA mixture is genetically amplified by the PCR method (Saiki, R., Scharb, S., Faloona, F. & Mullis, K., Science, 230: 1350-1354 (1985)). The PCR method is preferably RT-PCR using as a template cDNAs prepared from mRNAs using a reverse transcriptase, see Choi, Y. et al., Proc. Natl. Acad. Sci. USA, 86: 8941-8945 (1989). In the present invention, it is preferred to use as a primer the DNA fragment encoding the V region of the β chain and the DNA fragment encoding the C region of the β chain, in T-cell receptors.

More specifically, it is preferred to chemically synthesize oligonucleotides of about 20 bp encoding a part of subtypes of Vβ₁, Vβ₂, Vβ₃, Vβ₄, Vβ₅, Vβ₆, Vβ₇, Vβ₈, Vβ₉, Vβ₁₀, Vβ₁₁, Vβ₁₂, Vβ₁₃, Vβ₁₄, Vβ₁₅, Vβ₁₆, Vβ₁₇, Vβ₁₈, Vβ₁₉ and Vβ₂₀ in the V region of β chain (Yongwon Choi et al., Proc. Natl. Acad. Sci. USA, 86: 8941-8945 (1989)) and use the oligonucleotide as one of the primers. Subspecies of these subtypes, e.g., Vβ_{5.1}, Vβ_{5.2}, Vβ_{6.1}, Vβ_{13.1}, Vβ_{13.2}, etc. may also be employed in place of the subtypes described above. Preferably, an oligonucleotide of about 20 bp which encodes a part of the C region of β chain is used as one of the other primers. The primer may also be the one previously labeled with a radioisotope or biotin.

cDNAs are amplified by the PCR method in a conventional manner. More specifically, cDNAs are heated to about 94°C thereby to separate two strands of cDNAs. Annealing to the primers described above is then performed at a temperature of about 60°C followed by synthesis of the complementary chain at a temperature of about 72°C using DNA polymerase isolated from thermophilic bacteria Thermus aquaticus or thermophilic DNA polymerase having similar properties and a reaction solution containing four (4) kinds of deoxynucleotide-5'-triphosphates. The genes are amplified by repeating the procedure in, e.g., 35 cycles.

Thus, the genes having the same chain length which encode the V, D, J and C regions of β chain in the T-cell receptors are amplified. The genes are further analyzed using the SSCP technique (Orita, M. et al., Genomics, 5: 874-879 (1989); Orita, M., Proc. Natl. Acad. Sci. USA, 86: 2766 (1989)).

The SSCP method comprises separating double stranded DNA fragments to single strands and then performing electrophoresis on a non-denatured urea-free polyacrylamide gel, whereby DNA fragments having the same length are made further separable by the difference in its higher-order structure. The higher-order structure of DNA fragments is diversified even by a single nucleotide mutation so that different DNA fragments based on the single nucleotide mutation can be discriminated from each other.

Analysis by the SSCP technique is performed preferably by electrophoresis on a glycerol-added polyacrylamide gel having a low crosslinking degree. More specifically, it is preferred to use such a gel containing about 10% glycerol and having a N,N'-methylenebisacryl-amide/acrylamide ratio of approximately 1 : 49. After electrophoresis, where the genes amplified by the PCR method using a primer labeled with a radioisotope is employed, analysis of the genes may be performed by drying the gel and preparing an autoradiogram. In the case of using the genes amplified by the PCR method using a biotinated primer, analysis of the genes may be made by incubation together with a chemiluminescent system comprising streptavidin, biotinated alkaline phosphatase and a chemiluminescent substrate, or with a color-forming system comprising a color-forming substrate.

Where an unlabeled primer is employed in gene amplification by the PCR method, the genes may be analyzed by subjecting the amplified genes to analysis using the SSCP technique, electrophoresis transferring to membrane and then hybridization using a probe labeled with radioisotope or biotin. Representative examples of the probe which can be used for the above purpose are those obtained by chemically synthesize oligonucleotides of about 20 bp encoding a part of subtypes of Jβ1.1, Jβ1.2, Jβ1.3, Jβ1.4, Jβ1.5, Jβ1.6, Jβ2.1, Jβ2.2, Jβ2.3, Jβ2.4, Jβ2.5, Jβ2.6, Jβ2.7, etc. in the J region of β chain in T-cell receptors (Barry Toyonaga et al., Proc. Natl. Acad. Sci. USA, 82: 8624-8628 (1985)) and then, e.g., biotinating the 5' end. By hybridization using these probes, the genes specifically expressed in the J region of β chain can be detected.

Alternatively, there may be used a probe obtained by chemically synthesizing about 20 bp oligonucleotide encoding a part of the C region in T-cell receptors and then, e.g., biotinating the 5' end.

Where hybridization is performed using a probe labeled with radioisotope, the genes can be analyzed by preparing an autoradiogram. In the case of using a biotinated primer, the genes may be analyzed by incubation together with a chemiluminescent system comprising streptavidin, biotinated alkaline phosphatase and a chemiluminescent substrate, or with a color-forming system comprising a color-forming substrate.

As stated hereinabove, the genes different in terms of the D and J regions of β chain in T-cell receptors can be separated from each other. Therefore, the presence of a gene specific to T cell clones accumulated in a test sample can be detected by detecting the thus separated genes.

Hereinafter the present invention is described in more detail by referring to the examples.

### Example 1

### 1) Isolation of peripheral blood lymphocytes from a healthy individual

Peripheral blood (8 ml) was collected from a healthy individual by adding heparin thereto and lymphocytes were isolated from the blood in 5 × 10⁶ by Ficoll High Paque high density centrifugation.

### 2) Isolation of RNAs

RNAs were isolated from the lymphocytes isolated from 1) above according to a modification of the AGPC method (Chomczynski, P. & Sacchi, N., Anal. Biochem., 162:156-159 (1987)). That is, 0.5 ml of Solution D (250 g of guanidium thiocyanate, 17.6 ml of 0.75 M sodium citrate, pH 7.0, 26.4 ml of 10% sarcosyl, and 360 µl/50 ml of 2-mercaptoethanol) was added to lymphocyte pellets. After the mixture was well blended, 50 µl of 2 M sodium acetate (pH 4.0) was added to the mixture and 500 µl of phenol was further added thereto. The mixture was well blended with one minute interval, which was performed for 5 minutes.

Then 100 µl of chloroform was added to the mixture. After blending them well for a minute, the mixture was allowed to stand at 4°C for 15 minutes.

Next, the mixture was centrifuged at 15,000 rpm for 20 minutes. After 500 µl of isopropanol was added to the supernatant, the mixture was allowed to stand at -20°C for an hour. By centrifugation at 15,000 rpm for 20 minutes, the supernatant was removed to obtain RNA pellets.

The pellets were dissolved in 700 µl of Solution D and 700 µl of isopropanol was further added to the solution. After thoroughly mixing them, the resulting mixture was allowed to stand at -20°C for an hour followed by centrifugation at 5,000 rpm for 20 minutes. The thus obtained RNA pellets were washed twice with 75% ethanol.

Thus 10 µg of RNAs were obtained.

The purity and concentration of RNAs were assayed in terms of agarose gel and absorbance.

### 3) Synthesis of cDNAs

To 10 µg of the RNA pellets obtained in 2) above was added 400 µl of TE buffer (10 mM Tris-hydrochloride buffer, pH 7.5 : 1 mM EDTA) to dissolve the pellets. Then 40 µl of 3 M sodium acetate, pH 5.2 and 1 ml of ethanol were added to the solution. After thoroughly mixing them, the mixture was allowed to stand at -80°C for an hour followed by centrifugation at 15,000 rpm for 20 minutes. The thus obtained RNA pellets were washed twice with 75% ethanol.

To the thus obtained RNA pellets was added 9.5 µl of purified water. The mixture was allowed to stand at 70°C for 4 minutes to dissolve the pellets. The solution was allowed to stand at 4°C. To the solution were added 4 µl of 5 × reverse transcriptase reaction solution, 2 µl of 0.1 M DTT, 1 µl of RNase inhibitor, 10 nmols of dNTP and 1.5 µl of random primer. Further 1 µl of reverse transcriptase was added to the mixture followed by incubation at 42°C for 2 hours. cDNAs were thus obtained.

### 4) Synthesis of RT-PCR primer

### a. Synthesis of primers in the Vβ (variable) region

Primers for PCR (polymerase chain reaction) having nucleotide sequences shown in Table 1 below were synthesized on 392 DNA SYNTHESIZER manufactured by Applied Biosystems Co.

**Table 1**

| Primer | 5' 3' Nucleotide Sequence |
|---|---|
| Vβ1 | GCACAACAGTTCCCTGACTTGCAC |
| Vβ2 | TCATCAACCATGCAAGCCTGACCT |
| Vβ3 | GTCTCTAGAGAGAAGAAGGAGCGC |
| Vβ4 | ACATATGAGAGTGGATTTGTCATT |
| Vβ5.1 | ATACTTCAGTGAGACACAGAGAAAC |
| Vβ5.2 | TTCCCTAACTATAGCTCTGAGCTG |
| Vβ6.1 | AGGCCTGAGGGATCCGTCTC |
| Vβ7 | CCTGAATGCCCCAACAGCTCTC |
| Vβ8 | ATTTACTTTAACAACAACGTTCCG |
| Vβ9 | CCTAAATCTCCAGACAAAGCTCAC |
| Vβ10 | CTCCAAAAACTCATCCTGTACCTT |
| Vβ11 | TCAACAGTCTCCAGAATAAGGACG |
| Vβ12 | AAAGGAGAAGTCTCAGAT |
| Vβ13.1 | CAAGGAGAAGTCCCCAAT |
| Vβ13.2 | GGTGAGGGTACAACTGCC |
| Vβ14 | GTCTCTCGAAAAGAGAAGAGGAAT |
| Vβ15 | AGTGTCTCTCGACAGGCACAGGCT |
| Vβ16 | AAAGAGTCTAAACAGGATGAGTCC |
| Vβ17 | CAGATAGTAAATGACTTTCAG |
| Vβ18 | GATGAGTCAGGAATGCCAAAGGAA |
| Vβ19 | CAATGCCCCAAGAACGCACCCTGC |
| Vβ20 | AGCTCTGAGGTGCCCCAGAATCTC |

### b. Synthesis of primers in the Cβ (constant) region

Following the procedures of a. described above, amino acid residue is further added to the 5' end to synthesize PCR primer having the following nucleotide sequence shown by formula (1): 
5′ 3′
TTC TGA TGG CTC AAA CAC (1)
The amino-added 5' end was biotinated and the biotinated primer was provided for use.

### 5) RT-PCR

Using the thus obtained cDNAs in 2) above, the cDNAs were amplified by RT-PCR (reverse transcriptase-polymerase chain reaction: Choi, Y. et al., Proc. Natl. Acad. Sci. USA, 86: 8941-8945 (1989)).

That is, the cDNAs (about 0.2 µg when converted into RNAs) were amplified with 50 µl of 0.5 unit Taq (heat-resistant DNA) polymerase manufactured by Cetus using 5 µl of 10 × Taq polymerase buffer (500 mM KCl, 100 mM Tris-hydrochloride, 15 mM MgCl₂, 1% Triton X-100) in the presence of 50 pmols each of the primers in the respective V regions shown in Table 1 above and the 5' end biotinated primers and in the presence of 10 nmols each of four (4) deoxynucleotide triphosphates. For this procedure, the reaction mixture was coated with 10 µl of mineral oil. PCR was then performed in a DNA thermal cycler (which is a temperature-programmable block) by treating the mixture at 94°C for 1.5 minute, 60°C for 2 minutes and 72°C for 3 minutes in 35 cycles to amplify the objective DNAs.

### 6) Electrophoresis of the amplified DNAs on non-denatured gel

The PCR products obtained in 1) to 5) above were analyzed using the SSCP technique reported by Orita, M., Suzuki, Y., Sekiya, T., Hayashi, K. et al., Genomics, 5:874-879 (1989).

The PCR products obtained in 1) to 5) above were diluted in a denatured solution (95% formamide, 10 mM EDTA, 0.1% bromophenol blue and 0.1% xylene cyanol) in a ratio of 1 : 20 and treated at 90°C for 2 minutes.

Two microliters of the dilutant was then loaded on a non-denatured polyacrylamide gel (4% acrylamide/bis (49 : 1); 5 ml of 10 × TBE (0.5 M Tris, 20 mM Na₂EDTA, 0.97 M boric acid), pH 8.3; 5% glycerol; 250 µl of 10% ammonium persulfate (APS); and 50 µl of N,N,N',N'-tetramethylenediamine (TEMED)) for electrophoresis at 35 W for 2 hours.

After the electrophoresis, the DNAs on the gel were transferred to Immobilon-S (Milligen/Biosearch) and detected by subsequent incubations of streptavidin, biotinated alkaline phosphatase and PPD (chemiluminescent substrate system: Plex™ Luminescent Kit, Millipore/Biosearch).

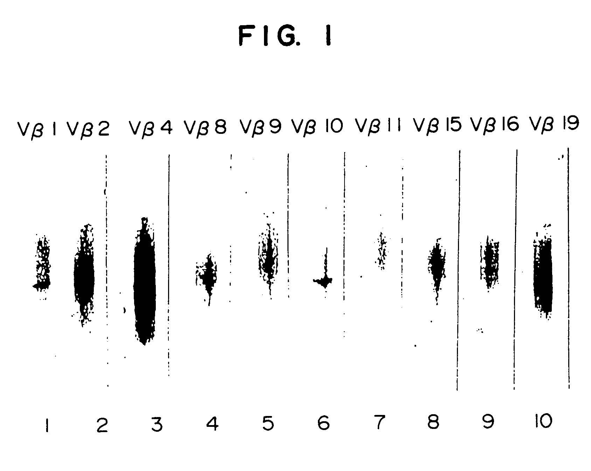

The results are shown in Fig. 1.

As is evident from Fig. 1, the amplified DNA was revealed generally as a smear in peripheral blood from healthy individuals. This suggests heterogeneous clonality of T-cell in this sample, not a state in which a particular clone has proliferated.

However, a single band was detected for Vβ₁ and Vβ₁₀, suggesting that there was some increase of clones in peripheral blood even from healthy individuals.

### Example 2

Human T cell tumor line HPB-ALL was proliferated in vitro. Using the HPB-ALL proliferated, the T-cell receptors were detected by the procedures of Example 1, 2), 3), 4), 5) and 6).

Similarly, detection was made by the procedures of Example 1, 4), 5) and 6), using cloned cDNA of Vβ_{6.1} as a template for PCR.

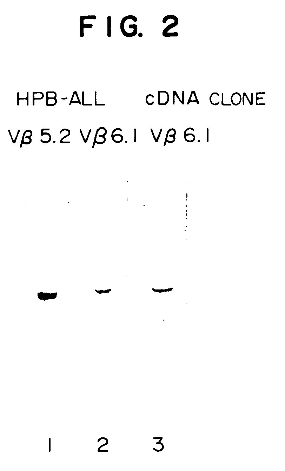

The results are shown in Fig. 2.

As shown in Fig. 2, a single band was obtained in analysis on T cells composed of a single clone.

### Example 3

RNAs of HPB-ALL isolated by the procedures of Example 1, 1) and 2) were mixed with RNAs isolated from healthy individual by the procedures of Example 1, 1) and 2) in a dilution system of 1 : 200 to 1 : 256000. Following the procedures of Example 1, 3), cDNAs were synthesized and detected by the procedures of Example 1, 4), 5) and 6).

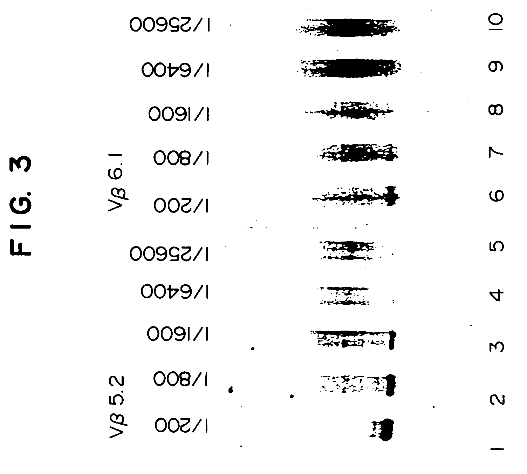

The results are shown in Fig. 3.

When the peripheral blood from healthy individual comprising a number of clones was mixed with the single T cell tumor line, a single band could be detected in the smear.

Study on detection limit reveals that accumulation of one clone in about 1000 clones makes the band detectable.

### Example 4

1) After 10 µg/ml (ConA) of mitogen, i.e., Concanavalin A (ConA) was added at the onset of incubation, 5 ml of peripheral blood lymphocytes (1 × 10⁶/ml were stimulated with ConA for 4 days. Similarly, phytohemagglutinin (PHA) was used for stimulation for 4 days with PHA diluted to 1 : 1000 at the onset of incubation. Similarly purified protein derivative (PPD) was used for stimulation for 4 days with 25 µg/ml of PPD at the onset of incubation.
2) Peripheral blood lymphocytes collected from healthy individual in 1) above were analyzed by the procedures of Example 1, 2), 3), 4), 5) and 6).

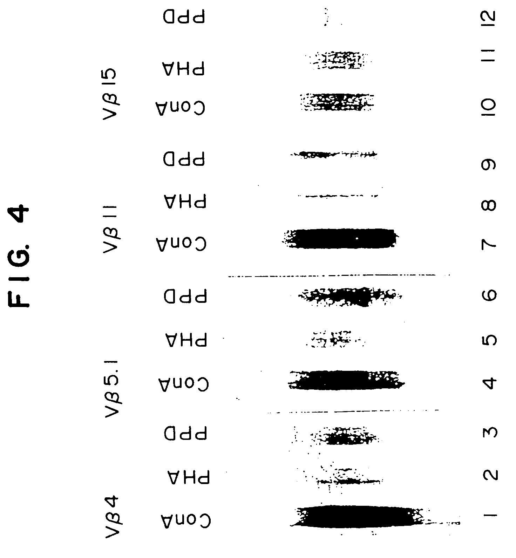

The results are shown in Fig. 4.

On the polyclonally stimulated peripheral blood lymphocytes from healthy individual with non-specific mitogen, the stimulation did not generate expansion of any particular clone but the DNAs were revealed as a smear. However, on the stimulation with specific antigen PPD, bands were detected in Vβ_{5.1} and Vβ₁₅. The results suggest that stimulation with a specific antigen may trigger the clonal expansion of certain T cells.

### Example 5

### 1) T-cell receptors infiltrated in the lesion of a patient with chronic rheumatoid arthritis (hereinafter referred to as RA patient)

### a. Peripheral blood from RA patient

According to 1) of Example 1, lymphocytes were isolated from peripheral blood of RA patient and then RNAs were isolated by the procedures of Example 1, 2).

### b. Synovial fluid (S.F.)

After lymphocytes were isolated from 20 ml of S.F. of the afflicted joint from the RA patient above according to the procedures of Example 1, 1), RNAs were isolated by the procedures of Example 1, 2).

### c. Synovial tissue (S.T.)

Two different S.T. sections of the same joint which is the inflammatory lesion were obtained from the RA patient and mixed with 0.5 ml of Solution D. After homogenization, RNAs were isolated by the procedures of Example 1, 2).

### 2) Following the procedures of Example 1, 3), 4), 5) and 6), the RNAs obtained in a, b and c above were analyzed.

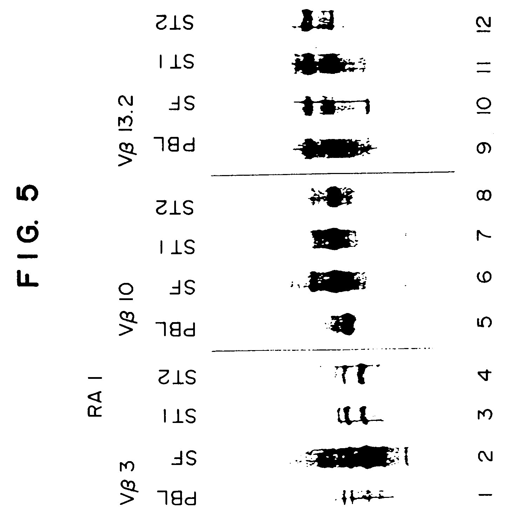

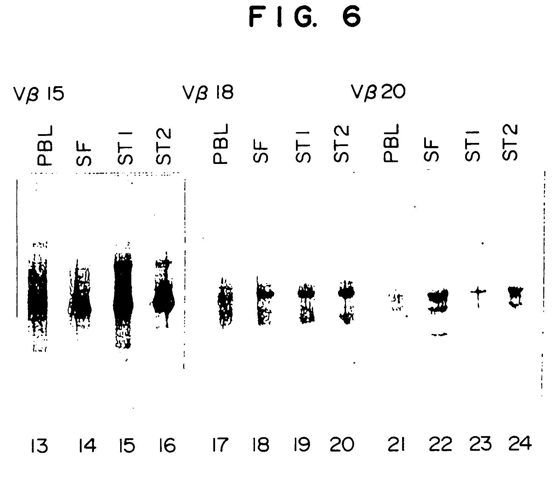

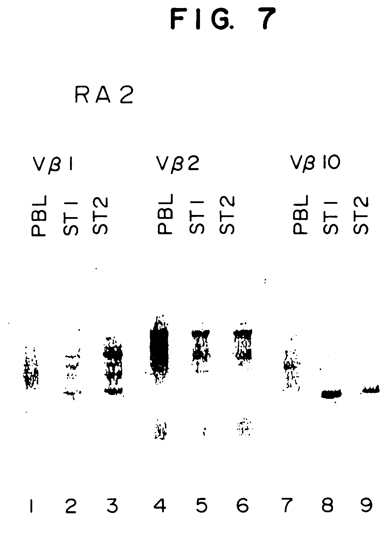

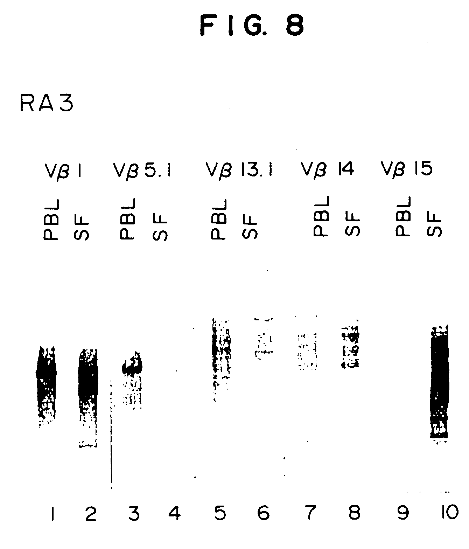

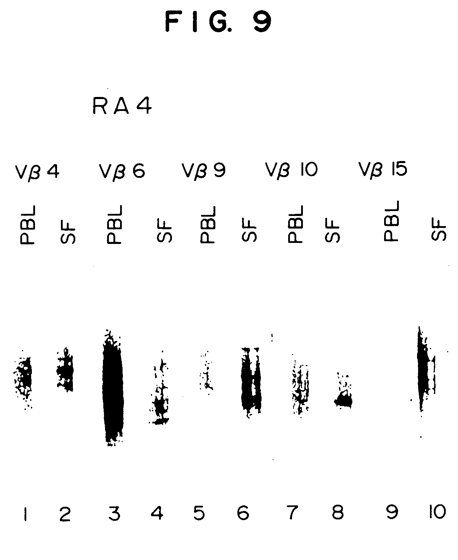

The results are shown in Figs. 5 through 9.

In the RA patient, clonal expansion was noted even in peripheral blood, as compared to healthy individuals.

Marked clonal accumulation was noted in the lymphocytes from synovial fluid and synovial tissues which are the focus of lesion.

The results therefore strongly suggest that the specific immune response to a certain antigen would take place at the joint.

### Example 6

### 1) T-cell receptors infiltrated in the lesion of a patient with uterus cancer (hereinafter referred to as U. Ca patient)

### a. Peripheral blood from U. Ca patient

According to 1) of Example 1, lymphocytes were isolated from peripheral blood of U. Ca patient and then RNAs were isolated by the procedures of Example 1, 2).

### b. Regional lymph node (LN)

Lymphocytes were isolated around the focus from the U. Ca patient above and mixed with 0.5 ml of Solution D. After homogenization of the mixture, RNAs were isolated by the procedures of Example 1, 2).

### c. Endometrium

The endometrium section containing tumor infiltrating lymphocytes (TIL) around the focus of the U. Ca patient above was mixed with 0.5 ml of Solution D. After homogenization, RNAs were isolated by the procedures of Example 1, 2).

### 2) Following the procedures of Example 1, 3), 4), 5) and 6), the RNAs obtained in a, b and c above were analyzed.

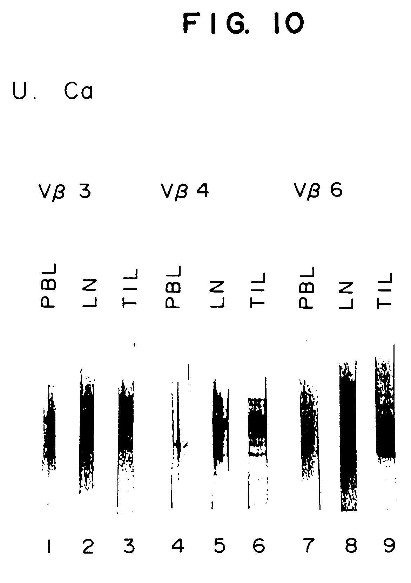

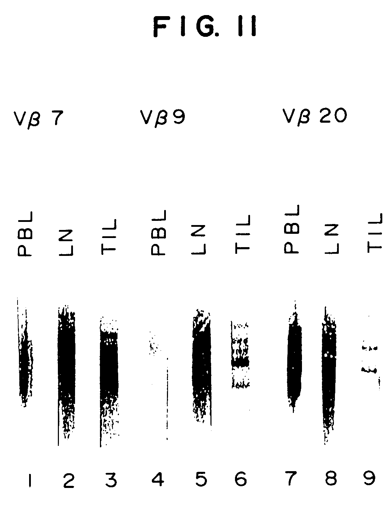

The results are shown in Figs. 10 and 11.

In the U. Ca patient, clonal accumulation was noted, indicating that tumor infiltrating T cells alone take place the specific immune response. Such clonal accumulation was not detected in the lymph nodes around the focus and the peripheral blood in which no metastasis was noted.

The results thus suggest that in such a case, immune responses to U. Ca can be expected only for the tumor infiltrating lymphocytes.

### Example 7

### 1) T-cell receptors infiltrated in the lesion of a patient with sarcoidosis

### a. Peripheral blood from the patient with sarcoidosis

According to 1) of Example 1, lymphocytes were isolated from peripheral blood of the patient with sarcoidosis and RNAs were then isolated by the procedures of Example 1, 2).

### b. Bronchoalveolar lavage (BAL)

After lymphocytes were isolated from the washing liquid of bronchoalveoli, which is the focus of the patient with sarcoidosis, with physiological saline according to the procedures of Example 1, 1), RNAs were prepared by the procedures of Example 1, 2).

### 2) Following the procedures of Example 1, 3), 4), 5) and 6), the RNAs obtained in a and b above were analyzed, respectively.

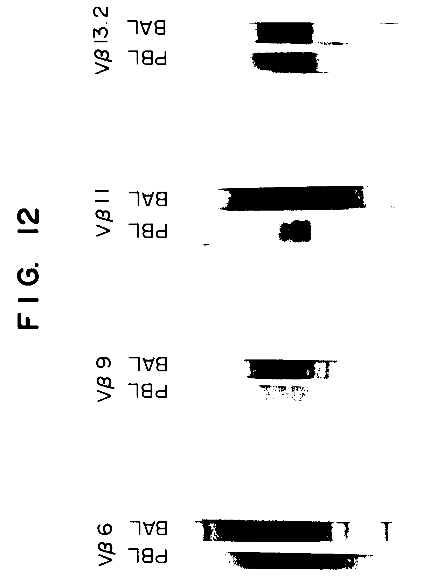

The results are shown in Fig. 12.

In the patient with sarcoidosis, clonal expansion was noted even in peripheral blood, as compared to healthy individuals.

Marked clonal accumulation was noted in the lymphocytes from the washing liquid of bronchoalveoli which is the focus of lesion.

The results therefore strongly suggest that the specific immune response to a certain antigen would take place in the bronchoalveoli.

### Example 8

### 1) T-cell receptors infiltrated in the lesion of a patient with hepatitis C

### a. Peripheral blood from the patient with hepatitis C

According to 1) of Example 1, lymphocytes were isolated from peripheral blood of the patient with hepatitis C and RNAs were then prepared by the procedures of Example 1, 2).

### b. Biopsy specimen

Liver biopsy sample which is the focus of the patient with hepatitis C was mixed with 0.5 ml of Solution D. After homogenization, RNAs were prepared by the procedures of Example 1, 2).

### 2) Following the procedures of Example 1, 3), 4), 5) and 6), the RNAs obtained in a and b above were analyzed, respectively.

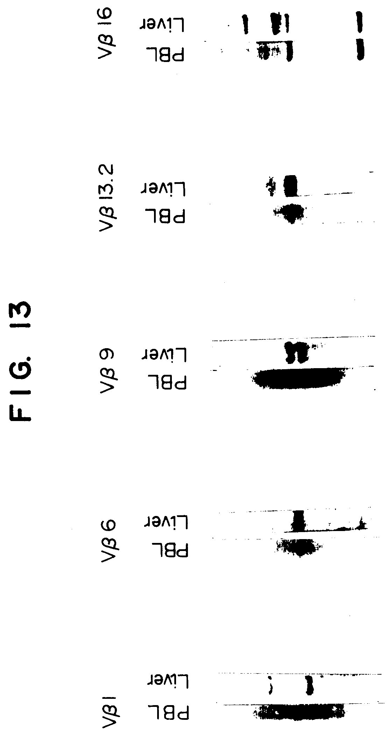

The results are shown in Fig. 13.

In the patient with hepatitis C, some clonal expansion was noted even in peripheral blood, as compared to healthy individuals.

Marked clonal accumulation was noted in the biopsy specimen which is the focus of lesion.

The results therefore strongly suggest that the specific immune response to a certain antigen would take place in the liver.

### Example 9

### Detection by hybridization technique:

### 1) T-cell receptors infiltrated in the lesion of RA patient by hybridization technique

### a. Peripheral blood from RA patient

According to 1) of Example 1, lymphocytes were isolated from peripheral blood of RA patient and RNAs were then prepared by the procedures of Example 1, 2).

### b. Synovial fluid (S.F.)

After lymphocytes were isolated from 20 ml of S.F. of the afflicted joint from the RA patient above according to the procedures of Example 1, 1), RNAs were prepared by the procedures of Example 1, 2).

### c. Synovial tissue (S.T.)

Two different S.T. sections of the same joint which is the inflammatory lesion were obtained from the RA patient and mixed with 0.5 ml of Solution D. After homogenization, RNAs were prepared by the procedures of Example 1, 2).

### 2) Following the procedures of Example 1, 3), cDNAs were synthesized.

### 3) RT-PCR

Using the thus obtained cDNAs in 2) above, the cDNAs were amplified by the RT-PCR technique (Choi, Y. et al., Proc. Natl. Acad. Sci. USA, 86: 8941-8945 (1989)). The procedures are performed in a manner similar to Example 1, 5). That is, the cDNAs (about 0.2 µg when converted into RNAs) were amplified with 50 µl of 0.5 unit Taq (heat-resistant DNA) polymerase manufactured by Cetus, using 10 × Taq polymerase buffer (500 mM KCl, 100 mM Tris-hydrochloride, 15 mM MgCl₂ and 1% Triton X-100) in the presence of 50 pmols each of the primers in the respective Vβ regions in Example 1, 4) and the 5' end non-biotinated primers having the same sequence as that of the C region primers shown in Table 1 above and in the presence of 100 nmols each of four (4) deoxynucleotide triphosphates. For this procedure, the reaction mixture was coated with 10 µl of mineral oil. PCR was then performed in a DNA thermal cycler (which is a temperature-programmable block) by treating the mixture at 94°C for 1.5 minute, 60°C for 2 minutes and 72°C for 3 minutes in 35 cycles to amplify the objective DNAs.

### 4) Electrophoresis of the amplified DNAs on non-denatured gel

The PCR products obtained in 1) to 3) above were electrophoresed using the SSCP technique.

After the electrophoresis, the DNAs on the gel were transferred to Immobilon-S (Milligen/Biosearch).

### 5) Synthesis of probe for hybridization

### a. Synthesis of probe in the Jβ (joining) region

A probe having an amino group at the 5' end in addition to the nucleotide sequence shown in Table 2 below was synthesized on 392 DNA Synthesizer manufactured by Applied Biosystems. The amino-added 5' end was biotinated and then provided for use.

### b. Synthesis of probe for the Cβ (constant) region

Following the procedure of a. above, a probe further added with an amino group at the 5' end and having a nucleotide sequence shown by the following formula (2):
5, - TTCTGATGGCTCAAACAC - 3'
was prepared to detect the Cβ region. The amino group-added 5' end was biotinated and then provided for use.

**Table 2**

| Primer | 5' 3' Nucleotide Sequence |
|---|---|
| Jβ1.1 | GCTTTCTTTGGACAAGGCA |
| Jβ1.2 | ATGGCTACACCTTCGGTTC |
| Jβ1.3 | TTTGGAGAGGGAAGTTGGCT |
| Jβ1.4 | TTTTTGGCAGTGGAACCC |
| Jβ1.5 | TGGTGATGGGACTCGACTCT |
| Jβ1.6 | ACTTTGGGAATGGGACCA |
| Jβ2.1 | AATGAGCAGTTCTTCGG |
| Jβ2.2 | TTTGGAGAAGGCTCTAGG |
| Jβ2.3 | AGTATTTTGGCCCAGGCA |
| Jβ2.4 | ACATTCAGTACTTCGGCG |
| Jβ2.5 | AGGCACGCGGCTCCTGGT |
| Jβ2.6 | AACGTCCTGACTTTCGGG |
| Jβ2.7 | GGGCACCAGGCTCACGGT |

### 6) Hybridization

The DNA-transferred Immobilon-S was allowed to stand at 42°C for 5 minutes in a prehybridization solution (5% SDS, 10% PEG, phosphate buffer, pH 7.2). Then, the probe for the Jβ or Cβ region prepared in 5) above was diluted in 1 pmol/ml of the prehybridization solution. The prehybridized Immobilon-S was treated with the diluted solution at 42°C for 30 minutes. After washing with a washing solution (0.5% SDS, phosphate buffer, pH 7.2), the DNAs were detected by incubations of streptavidin, biotinylated alkaline phosphatase and PPD (chemiluminescent substrate system: Plex™ Luminescent Kit, manufactured by Milligen/Biosearch).

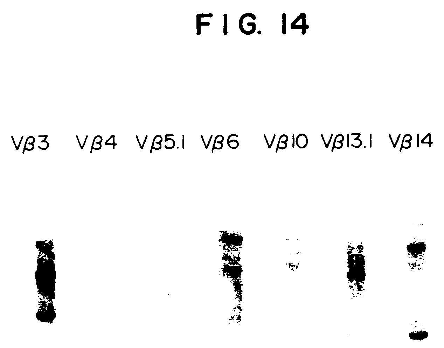

The results are shown in Fig. 14.

As is clear from Fig. 14, results similar to those detected using the biotinated primers were obtained also in the case of detection after hybridization with the probe for the Cβ region. In the peripheral blood from the RA patient, clonal expansion was noted as compared to healthy individual. Remarkable clonal accumulation was recognized at the junctional lesion (both in S.F. and in S.T.), as compared to healthy individual. The hybridization approach reveals that the detection sensitivity was comparable to that of Example 5.

Where hybridization was conducted using the probe for the Jβ region, a difference in frequency of using Jβ was noted, strongly suggesting that these accumulated clones would recognize a specific antigen.

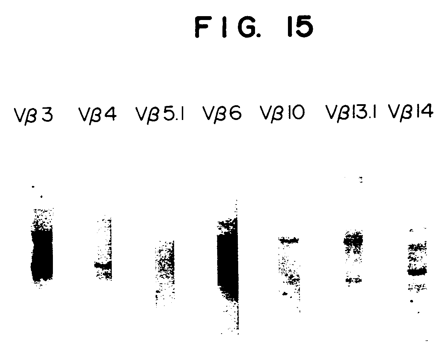

The results obtained by hybridization using the Jβ2.1 probe are shown in Fig. 15. As is clearly noted from Fig. 15, a specific clone expressing Jβ2.1 was detected.

### INDUSTRIAL APPLICABILITY

As is appreciated from the description above, the genes of T cell receptors specific to T cells accumulated in a test sample can be detected according to the method of the present invention. By applying the method of the present invention, T cell receptor genes capable of specifically recognizing pathogenic antigens associated with autoimmune disease such as chronic rheumatoid arthritis can be detected and monitored to analyze the D and J regions. Such analyses lead to clarify the antigen recognition site of T-cell receptors specifically expressed to the pathogenic antigens and to prepare vaccine for blocking the recognition site or administer antibodies to the recognition site. This enables to develop a specific treatment for suppressing the corresponding immune response only.

## Claims

1. A method for detecting the presence of T cell receptor genes which comprises:
preparing a cDNA mixture comprising T cell-derived cDNA from a test sample containing T cells;
subjecting the cDNA mixture to a polymerase chain reaction using as a primer a DNA fragment encoding the β chain of T-cell receptors to amplify the genes encoding the β chain of the T-cell receptors in the cDNA mixture; and then,
subjecting the thus amplified genes to analysis by a single strand conformation polymorphism technique to detect the presence of the T cell clone-specific genes accumulated in the test sample which encodes the β chain of the T-cell receptors.

2. A method according to claim 1, wherein said test sample is obtained from fluid or tissue of a patient suspected of abnormal immune response, a patient with viral disease, a patient with cancer or a patient with hepatitis.

3. A method according to claim 2, wherein a DNA fragment encoding the V region of β chain and a DNA fragment encoding the C region of β chain, in T-cell receptors are employed as said primer for PCR.

4. A method according to claim 3, wherein said DNA fragment encoding the V region of β chain in T-cell receptors used as the primer for PCR is a DNA fragment encoding the region selected from a subtype set of Vβ₁ to Vβ₂₀.

5. A method according to any one of claims 1 to 4, wherein analysis by the SSCP technique is performed on glycerol-added acrylamide having a low crosslinking degree.

6. A method according to any one of claims 1 to 5, wherein the presence of a T cell clone having a specific sequence in the DNA sequence encoding the D region and/or J region of β chain in the T-cell receptors and genes thereof are detected as genes specific to T cell clones accumulated in said test sample.

7. A method according to any one of claims 1 to 6, wherein analysis by the SSCP technique is performed by means of hybridization using as a probe a DNA fragment encoding the J region of β chain of the T cell receptors.
